# EUROPEAN PATENT SPECIFICATION

(11) **EP 2 495 331 B1**
(45) Date of publication and mention of the grant of the patent: **12.10.2016**
(21) Application number: 11789053.3
(22) Date of filing: 30.05.2011
(51) Int. Cl.: C12Q 1/18, C12N 1/20, C12R 1/32, C12M 1/34, C12M 1/38, G01N 33/569

(54) **TESTING METHOD FOR DRUG SENSITIVITY OF MYCOBACTERIUM TUBERCULOSIS, APPLICATION OF INDICATOR AND SOLID MEDIUM**
TESTVERFAHREN FÜR DIE ARZNEIMITTELEMPFINDLICHKEIT VON MYOBACTERIUM TUBERCULOSIS, ANWENDUNG DER ANZEIGE UND FESTES MEDIUM
PROCÉDÉ DE TEST DE LA SENSIBILITÉ DE MYCOBACTERIUM TUBERCULOSIS À UN MÉDICAMENT, APPLICATION D'UN INDICATEUR ET MILIEU SOLIDE

(30) Priority: 02.06.2010 CN 201010188846
(43) Date of publication of application: 05.09.2012
(73) Proprietor: Hunan-Tech New Medical Systems Co., Ltd, Hunan 410000 (CN)
(72) Inventor: PENG, Jun, Hunan 410000 (CN)
(74) Representative: Hryszkiewicz, Danuta
(86) International application number: PCT/CN2011/000917
(87) International publication number: WO 2011/150674

(56) References cited:
- CN-A- 1 298 023
- CN-A- 101 560 490
- CN-A- 102 010 886
- MISHRA BAIJAYANTI ET AL: "Direct drug susceptibility testing of Mycobacterium tuberculosis to primary anti-tubercular drugs by nitrate reductase assay", INDIAN JOURNAL OF PATHOLOGY & MICROBIOLOGY, vol. 52, no. 3, 12 August 2009 (2009-08-12), pages 343-344, XP055064510,
- B. VAN KLINGEREN ET AL: "Drug Susceptibility Testing of Mycobacterium tuberculosis Complex by Use of a High-Throughput, Reproducible, Absolute Concentration Method", JOURNAL OF CLINICAL MICROBIOLOGY, vol. 45, no. 8, 30 May 2007 (2007-05-30), pages 2662-2668, XP055064543, ISSN: 0095-1137, DOI: 10.1128/JCM.00244-07
- T. SCHABERG ET AL: "Rapid drug susceptibility testing of <I>Mycobacterium tuberculosis</I> using conventional solid media", EUROPEAN RESPIRATORY JOURNAL, vol. 8, no. 10, 1 October 1995 (1995-10-01), pages 1688-1693, XP055064669, ISSN: 0903-1936, DOI: 10.1183/09031936.95.08101688
- Y ZHANG ET AL: "Molecular basis for the exquisite sensitivity of Mycobacterium tuberculosis to isoniazid", PROC. NATL. ACAD. SCI. USA, vol. 93, 1 November 1996 (1996-11-01), pages 13212-13216, XP055064672,
- WITEBSKY F G ET AL: "Identification of mycobacteria by conventional methods.", CLINICS IN LABORATORY MEDICINE SEP 1996, vol. 16, no. 3, September 1996 (1996-09), pages 569-601, XP008162547, ISSN: 0272-2712
- BELANGER A E ET AL: "THE EMBAB GENES OF MYCOBACTERIUM AVIUM ENCODE AN ARABINOSYL TRANSFERASE INVOLVED IN CELL WALL ARABINAN BIOSYNTHESIS THAT IS THETARGET FOR THE ANTIMYCOBACTERIAL DRUG ETHAMBUTOL", PROCEEDINGS OF THE NATIONAL ACADEMY OF SCIENCES, NATIONAL ACADEMY OF SCIENCES, US, vol. 93, 1 October 1996 (1996-10-01), pages 11919-11924, XP002912066, ISSN: 0027-8424, DOI: 10.1073/PNAS.93.21.11919
- HEIFETS L B ET AL: "Drug susceptibility testing of Mycobacterium tuberculosis: a neglected problem at the turn of the century", INTERNATIONAL JOURNAL OF TUBERCULOSIS AND LUNG DISEASE, INTERNATIONAL UNION AGAINST TUBERCULOSIS AND LUNG DISEASE (I U A T L D), FRANCE, vol. 3, no. 7, 1 July 1999 (1999-07-01), pages 564-581, XP009037738, ISSN: 1027-3719
- A DE LOGU ET AL: "Comparison of Two Rapid Colorimetric Methods for Determining Resistance of Mycobacterium tuberculosis to Rifampin, Isoniazid, and Streptomycin in Liquid Medium", EUR J CLIN MICROBIOL INFECT DIS, vol. 20, 1 February 2001 (2001-02-01), pages 33-39, XP055064583,
- WAYNE L G ET AL: "Preparation of tuberculosis susceptibility testing mediums by means of impregnated disks", AMERICAN JOURNAL OF CLINICAL PATHOLOGY, AMERICAN SOCIETY FOR CLINICAL PATHOLOGY, US, vol. 45, no. 6, 1 June 1966 (1966-06-01), pages 769-771, XP008162588, ISSN: 0002-9173
- ZUO FENG-QIN ET AL.: 'Characterization of extracellular products from vibrio alginolyticus isolated from maricultured fish' ACTA HYDROBIOLOGICA SINICA vol. 30, no. 5, 30 September 2006, pages 554 - 556, XP008158320
- HENRY D. ISENBERG ET AL.: 'Collaborative Feasibility Study of a Biphasic System (Roche Septi-Chek AFB) for Rapid Detection and Isolation of Mycobacteria' JOURNAL OF CLINICAL MICROBIOLOGY vol. 29, no. 8, 31 August 1991, pages 1719 - 1722, XP008158984
- R.KNOX ET AL.: 'Semi-solid Agar media for rapid drug sensitivity Tests on Cultures of Mycobacterium tuberculosis' J.GEN.MICROBIOL. vol. 16, 31 December 1956, pages 647 - 659, XP008159000
- CHIYOJI ABE ET AL.: 'Comparison of MB-Check, BACTEC, and Egg-Based Media for Recovery of Mycobacteria' JOURNAL OF CLINICAL MICROBIOLOGY vol. 30, no. 4, 30 April 1992, pages 878 - 881, XP008158953
- HU ZHONGYI: 'Rapid testing method and evaluation for drug tolerence of mycobacterium tuberculosis' CHINESE JOURNAL OF TUBERCULOSIS AND RESPIRATORY DISEASES vol. 26, no. 2, 28 February 2003, pages 107 - 109, XP008158999
- D M Yajko ET AL: "Colorimetric method for determining MICs of antimicrobial agents for Mycobacterium tuberculosis", Journal of Clinical Microbiology, 1 September 1995 (1995-09-01), pages 2324-2327, XP055163732, UNITED STATES Retrieved from the Internet: URL:http://jcm.asm.org/content/33/9/2324.a bstract

## Description

The invention relates to the field of laboratory medicine for testing the drug resistance of *mycobacterium tuberculosis,* and more particularly to a method for testing drug sensitivity of *mycobacterium tuberculosis,* an application of an indicator used for the method for testing drug sensitivity of *mycobacterium tuberculosis.* Disclosed is a solid medium as well as a heating thermostat unit and a culture box for the method for testing drug sensitivity of *mycobacterium tuberculosis.*

The drug resistance of tuberculosis is a developing public health issue, which poses a serious threat to the control of tuberculosis. Chinese Academy of Engineering academician Zhong Nanshan said: there are almost 4.5 million patients with active tuberculosis in China, the number of bacteria carriers is up to 550 million, and the probability of susceptibility will be 10% in the life of a bacteria carrier. In April 2009, Chinese Health Minister Chen Zhu reported the case of drug-resistant tuberculosis in China in the ministerial meeting of "Multi-Drug Resistant TB and Extensively Drug Resistant TB high-burden country" held in Beijing. The sample surveys from the Chinese Center for Disease Control and Prevention revealed that in tuberculosis patients in China, the incidence rate of multi-drug resistant TB is 8.32%, and the total number of patients with multi-drug resistant TB is about 120,000, ranking second in the world; the incidence rate of extensively drug resistant TB is 0.68%, the total number of patients with extensively drug resistant TB is about 10,000, and its harm is far more than AIDS. A drug sensitivity test is important for detection of drug resistance of *mycobacterium tuberculosis.* The prior method for testing drug sensitivity of *mycobacterium tuberculosis* generally includes a paper strip test method and a concentration test method. The paper strip test method is used for determining the sensitivity of *mycobacterium tuberculosis* to drugs through observing a drug inhibition zone formed on a medicinal paper strip stuck on the surface of the solid medium. The concentration test method is used for determining the sensitivity of *mycobacterium tuberculosis* to drugs through observing and detecting the growth conditions of *mycobacterium tuberculosis* in test containers configured according to different drugs and different drug concentrations. In both the paper strip test method and the concentration test method, the whole drug sensitivity test process generally includes three stages: specimen pretreatment, multiplication or separation culture, and drug sensitivity test. In the prior paper strip test method, the stage of sample pretreatment includes the following steps:
1. Putting a specimen through a suction tube into a test container;
2. Adding digestive fluid in the test container, digesting the mucus in the specimen, and fully exposing the mucus in the specimen as well as the impurity-coated bacteria; and
3. After centrifugal separation, dumping supernatant fluid out to obtain enriched specimens; performing multiplication or separation culture after the enriched specimens are obtained.

The stage of multiplication or separation culture includes the following steps:
4. Preparing a smaller number of the enriched specimens into a strong bacteria liquid; and
5. Inoculating the strong bacteria liquid on the solid medium, for example, multiplication or separation culture on the L-J medium, usually cultivating the strong bacteria liquid in a incubator for about one month at the temperature of 37°C, and then cultivating individual bacteria into bacterial colonies; entering the stage of drug sensitivity test after the bacterial colonies are obtained.

The stage of drug sensitivity test includes the following steps:
6. Taking a small amount of bacterial colonies (1-3 bacterial colonies are generally taken), and dissolving and dispersing the bacterial colonies to obtain a strong bacteria liquid;
7. Inoculating the strong bacteria liquid on the solid medium, for example, inoculated on the surface of the agar medium;
8. Sticking a medicinal paper strip onto the surface of the solid medium;
9. Cultivating the strong bacteria liquid in an incubator with temperature of 37°C for about one month, and then observing the drug inhibition zone for determining the sensitivity of *mycobacterium tuberculosis* to drugs.

The paper strip test method is disclosed by, for example, Zhang, Y., et al., Molecular basis for the exquisite sensitivity of mycobacterium tuberculosis to isoniazid. Proc. Natl. Acad. Sci. USA, vol. 93 (1996): 13212-13216. The method for testing drug sensitivity of *mycobacterium smegmatis* of Zhang includes: a) adding *mycobacterium smegmatis* to Middlebrook 7H9 medium to obtain liquid specimen; b) culturing the liquid specimen for 3 or 4 days; c) mixing the liquid specimen with soft 7H10 agar to obtain a mixture and solidifying the mixture to obtain a solid specimen; d) placing paper discs impregnated with isonicotinic acid hydrazide (INH) on top of the solid specimen; and e) observing drug inhibition zones on the solid specimen after overnight incubation.

The prior paper strip test method has the advantages of convenience in operation, simple equipment and small investment; however, it also has the disadvantages that (1) The time taken in the whole test is too long: the stage of multiplication or separation culture takes about one month, the stage of drug sensitivity test takes about one month, and the whole test takes about two months. If the drug therapy is adopted after the drug sensitivity test, the treatment time will be seriously affected. The drug therapy adopted under the condition without clinical drug sensitivity test may cause negative effects: firstly, the serious consequence of drug resistance of *mycobacterium tuberculosis* may be caused; secondly, the good therapeutic effect for the tuberculosis patients suffering from drug-resistance *mycobacterium tuberculosis* is difficult to achieve. (2) The operation is not safe. The artificial inoculation shall be carried out twice in the whole test. Because the *mycobacterium tuberculosis* is infectious, potential safety hazards may be caused to operators, and the risk of pollution to the environment may be also caused.

In the prior concentration test method, the solid medium is adopted at the stage of multiplication culture. The steps of the two stages of specimen pretreatment and multiplication or separation culture are same as those in the prior paper strip test method, but the steps of the stage of drug sensitivity test are different from those in the prior paper strip test method, specifically, taking one or more bacterial colonies obtained through multiplication or separation culture from the solid medium; dissolving and dispersing the bacterial colonies to obtain a bacterial suspension; pouring the bacterial suspension into a plurality of test containers configured according to different drugs and different concentrations to be prepared into comparison test specimens; observing and detecting the growth conditions of *mycobacterium tuberculosis* in the test containers for determining the sensitivity of *mycobacterium tuberculosis* to drugs. The concentration test method has the advantages of simple equipment and small investment; however, it also has the disadvantages that (1) The required time is long, because the method and steps of the stage of multiplication or separation culture are same as those in the prior paper strip test method, the time only required at the stage of multiplication or separation culture takes about one month; (2) The operation is complicated and unsafe, and wrong test results are easily caused. A plurality of test containers are required to be configured, and the configuration process is cumbersome and unsafe, thus potential safety hazards may be caused to operators, and the risk of pollution to the environment may be also caused; the test containers are polluted by other bacteria more easily during the configuration, so wrong test results may be caused.

In addition, a method of identifying the drug sensitivity using instruments is also disclosed, for example, a BACTEC-TB460 system and a BACTEC 960 system are used. The method has the advantages of simple operation and short time of test process, wherein the mean detection time is 14.4 days, and the shortest time is about 10 days. The main disadvantages of the method are as follows: the instrument is high in price and high in vestment; the use cost is high, firstly, the use cost of the instruments is high, and secondly, because a plurality of comparison test specimens are required, the cost of reagent is higher; its high cost is difficult to bear for ordinary hospitals, thus the method is not popularized easily.

From the detailed analysis for the prior method for testing drug sensitivity of *mycobacterium tuberculosis,* it can be seen that the prior paper strip test method and concentration test method have the following disadvantages: 1. too long time required in the whole test process; 2. complicated operation; 3. unsafe operation; 4. detection failures are easily caused. The method of identifying the drug sensitivity using instruments is high in vestment, high in use cost, and difficult in popularization.

One objective of the invention is to provide a method for testing drug sensitivity of *mycobacterium tuberculosis,* an application of an indicator, and a solid medium that have less investment, low use cost, and safe and convenient use.

To achieve the objective of the invention, a method for testing drug sensitivity of *mycobacterium tuberculosis* comprises the following steps:
(1) Pre-treating target specimens to obtain enriched specimens;
(2) Adding a liquid medium to the enriched specimens to obtain liquid culture specimens, and performing enriched culture for 0-7 days;
(3) Heating and melting a solid medium to obtain a liquefied solid medium, and reducing the temperature of the liquefied solid medium to the corresponding culture temperature of the liquid culture specimens or room temperature, the solid medium having a melting temperature of 50-95°C and a solidification temperature of 25-45°C, the solid medium comprising vitelline, soluble starch, L-casein, phenol red, agar or agarose, bacteriostat, and urea;
(4) Mixing the liquid culture specimens with the liquefied solid medium to obtain liquid specimens for testing of drug sensitivity;
(5) Solidifying the liquid specimens for testing of drug sensitivity to yield solid specimens for testing of drug sensitivity with antibiotic discs;
(6) Cultivating the solid specimens for testing of drug sensitivity until the solid specimens turn pink or light yellow;
(7) Dripping an indicator to the solid specimens when the sold specimens turn pink or light yellow in (6), observing a drug inhibition zone through a visible color change of the indicator and determining the drug sensitivity of *mycobacterium tuberculosis.*

In the method for testing drug sensitivity of *mycobacterium tuberculosis,* the indicator is a *mycobacterium tuberculosis* growth indicator or biochemical reaction indicator.

In the method for testing drug sensitivity of *mycobacterium tuberculosis,* the indicator is a tellurite, a mixture of a tellurite and urea, urea, 3-(4,5-dimethylthiazol-2-yl)-2,5-diphenyltetrazolium bromide (MTT), 2,3-bis-(2-methoxy-4-nitro-5-sulfophenyl)-2H-tetrazolium-5-carboxanilide (XTT) or Alamar-Blue.

In the method for testing drug sensitivity of *mycobacterium tuberculosis,* the indicator tellurite is dripped into the solid drug sensitivity test specimen.

In the step (3), after the liquefied solid medium is prepared, reducing the temperature to the corresponding culture temperature of the liquid culture specimens or room temperature means that the temperature of the liquefied solid medium is reduced to the same or similar temperature relative to the liquid culture specimen under the condition of enriched culture of the liquid culture specimen; if the enriched culture is not performed, the temperature will be reduced to room temperature, but the solid medium still exists in a liquid state.

The invention further provides a use of an indicator for a method for testing drug sensitivity of *mycobacterium tuberculosis,* the indicator is a tellurite or a mixture of tellurite and urea; the indicator tellurite is dripped into solid specimens for testing of drug sensitivity.

The tellurite is sodium tellurite or potassium tellurite.

A mixture ratio between the solid medium and liquid medium in the liquid drug sensitivity test specimen is adding a part of solid medium in every 100 mL of liquid medium. Each part of solid medium comprises 50-100 mL of vitelline fluid, bottom, and an open upper end, wherein a ruler is arranged at the bottom of the box body, and a drug-sensitive paper strip is stuck on the upper side surface of the bottom of the box body.

In the culture box, six drug-sensitive paper strips are stuck on the upper side surface of the bottom of the box body.

The drug sensitivity test method of the invention is compared with the prior paper strip test method and concentration test method. The causes of short time taken in the whole test process are as follows:
1. Full utilization of to-be-tested *mycobacterium tuberculosis* in the provided specimen. Most of the enriched *mycobacterium tuberculosis* in the provided specimens is used for drug sensitivity test, the to-be-tested *mycobacterium tuberculosis* in the specimen is fully utilized, the initial bacteria amount in the drug sensitivity test is large, and the time of enriched culture is reduced, so that the whole test time is saved.
2. Short time at the stage of enriched culture. Because the enriched culture of the invention is carried out in the liquid medium, comparatively speaking, the multiplication rate of bacteria in the liquid medium is higher than that of bacteria in the solid medium; furthermore, the to-be-tested *mycobacterium tuberculosis* in the provided specimen is fully utilized, and the initial bacteria amount before enriched culture is large, so that the time at the stage of multiplication culture is short and only 7 days at most. When the detection result about the condition of a tuberculosis patient is ++++, a large quantity of *mycobacterium tuberculosis* used in the drug sensitivity test specimen is provided, thus the multiplication culture can be saved during the drug sensitivity test.
3. Short time at the stage of drug sensitivity test. The liquid culture specimen after multiplication culture is mixed with the liquefied solid medium in the invention, the solid drug sensitivity test specimen provided with drug sensitive paper strip is prepared by a tilt-pour process, and all of the *mycobacterium tuberculosis* after multiplication culture is used for drug sensitivity test, so that the initial bacteria amount in the drug sensitivity test is large, the drug inhibition zone is formed more easily, the inhibition zone can be visually inspected under the indication action of the indicator, the stage of drug sensitivity test only takes 5-15 days, and compared with the prior paper strip test method, the required time is shorter.
4. Use of quick colored indicator. Because the tellurite is used as the indicator in the invention, through repeated trials from the inventor, it proves that the bacterial colonies formed in the solid drug sensitivity test specimen are black or dark gray within 2-48 hours.
5. Use of previous indicator. The urea is added in the solid medium in the invention, the *mycobacterium tuberculosis* further generates urease for decomposing urea, and the solid drug sensitivity test specimen is pink or light yellow, so that the growth conditions of *mycobacterium tuberculosis* in the solid drug sensitivity test specimen is preliminarily judged at the stage of drug sensitivity test by observing the color of the solid drug sensitivity test specimen. When the solid drug sensitivity test specimen is pink or light yellow, it shows that the number of *mycobacterium tuberculosis* in the solid drug sensitivity test specimen reaches the number required for the formation of the inhibition zone, and the obvious inhibition zone can be formed within 2-48 hours after the telluride indicator is dripped, so that the time at the stage of drug sensitivity test can be shortened. Through repeated trials from the inventor, it proves that when the method for testing drug sensitivity of *mycobacterium tuberculosis* is adopted, the mean detection time is about 10 days. In conclusion, compared with the prior paper strip test method and concentration test method, the drug sensitivity test method achieves the objective of short time in the test process.

The reasons why the drug sensitivity test method is less in vestment and low in use cost are that the equipment required for implementing the invention is only required to be equipped with a centrifugal device, incubator, heating thermostat unit, etc; the centrifugal device, incubator, heating thermostat unit are simple to manufacture and low in production cost, thus the equipment investment required for completing the whole test is less; the non-returnable test container as well as the culture medium, indicator and other reagents are fewer and low in cost, and required to be used in the implementation of the invention in a matched manner, and the instrument for the invention is less in vestment and low in use cost, so that the use cost of the invention is further reduced. Therefore, compared with the method of identifying the drug sensitivity using the instruments, the drug sensitivity test method effectively achieves the objectives of less investment and low use cost.

The reasons why the drug sensitivity test method is safe and convenient to use are that both the enrichment of the specimen to be tested and the result interpretation can be performed in the non-returnable closed container, the other equipment are not polluted, the unified sterilization treatment is carried out after the completion of operation, and the manual operation procedures during the whole test process are fewer and simple, so that the influence of *mycobacterium tuberculosis* to working personnel and the environment is reduced, and the biological safety of test is greatly enhanced; the possibility of suffering from pollution from other bacteria is reduced, and the wrong test results can be effectively reduced. Therefore, compared with the prior paper test method and concentration test method, the drug sensitivity test method can effectively achieve the objective of safe and convenient use.

The following drawings and embodiments aim to serve as the further description for a method for testing drug sensitivity of *mycobacterium tuberculosis,* an application of an indicator as well as a solid medium in the invention.
FIG. 1 is a top view of a heating thermostat unit in the invention;
FIG. 2 is a sectional view taken from line A-A of a heating thermostat unit shown in FIG. 1;
FIG. 3 is a top view of a culture box without a box cover in the invention; and
FIG. 4 is a sectional view of a culture box shown in FIG. 3.

1. shell; 2. clamping groove of inverted plate; 3. third hole for clamping mixing cup; 4. heat-conduction block; 5. heat-insulation wall; 6. first hole for clamping high-temperature cup; 7. control panel; 8. second hole for clamping constant-temperature cup; 9. electric heating device; 10. box body; 11. drug-sensitive paper strip; 12. ruler; 13. box cover.

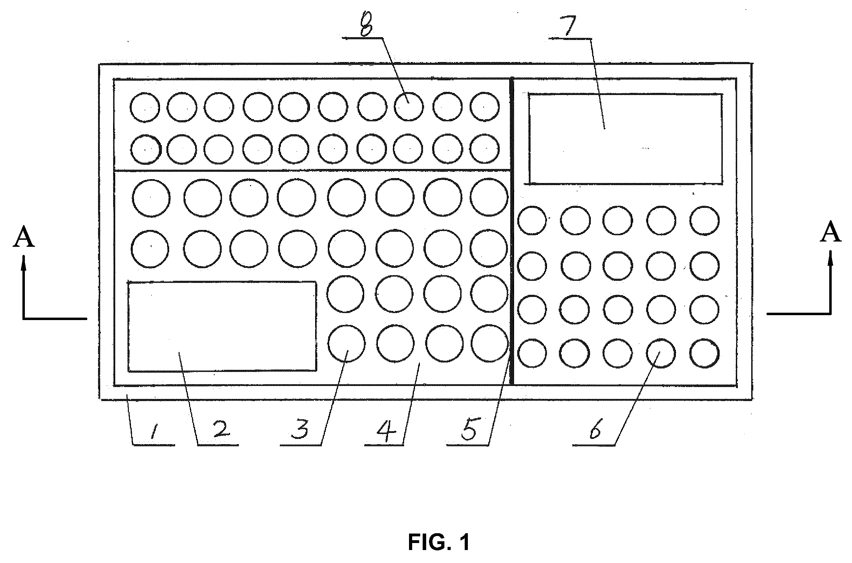

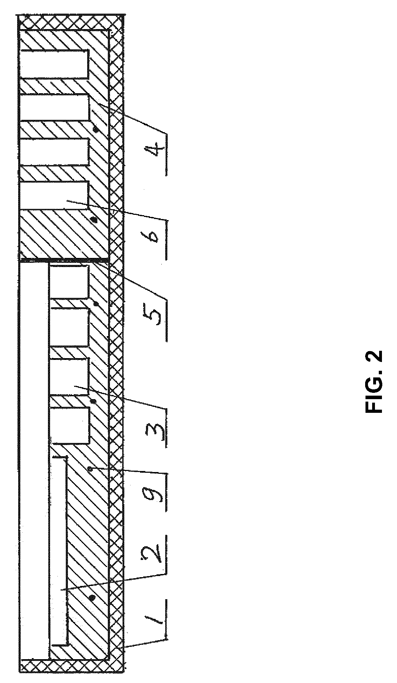

A heating thermostat unit is shown in FIG. 1 and FIG. 2. A heat-conduction block 4 is installed on a shell 1, and provided with a high-temperature zone and a constant-temperature zone. The temperature of the high-temperature zone is adjustable within the range of 50-95° C, and the optimal temperature is 90° C. The temperature of the constant-temperature zone is adjustable within the range of 25-45° C, and the optimal temperature is 42° C. A first hole for clamping a high-temperature cup 6 and a control panel 7 are arranged in the high-temperature zone. A constant-temperature balancing zone and a mixing zone are arranged in the constant-temperature zone. A second hole for clamping a constant-temperature cup 8 is formed in the constant-temperature balancing zone. A third hole for clamping a mixing cup 3 and a clamping groove of an inverted plate 2 are formed in the mixing zone respectively. A heat-insulation wall 5 is arranged between the high-temperature zone and the constant-temperature zone. An electric heating device 9 is arranged in the heat-conduction block 4. A temperature control device is arranged in the shell 1.

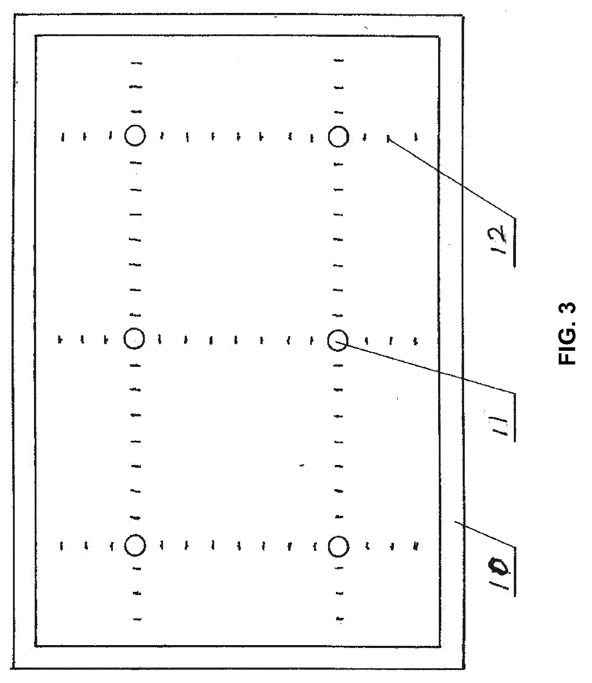

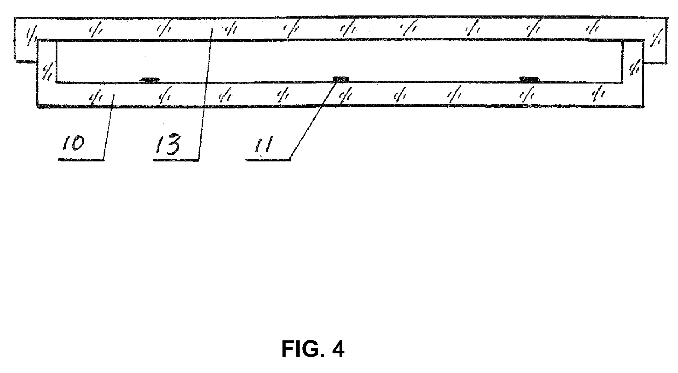

A culture box is shown in FIG. 3 and FIG. 4. A box body 10 adopts a structure with a closed circumferential wall, a closed bottom, and an open upper end, wherein a ruler 12 is arranged at the bottom of the box body 10, drug-sensitive paper strips 11 are stuck on the upper side surface of the bottom of the box body 10, and a box cover 13 is matched at the open end of the box body 10.

The embodiments for a method for testing drug sensitivity of *mycobacterium tuberculosis,* the application of an indicator as well as a solid medium in the invention are given below.

In the culture box of the embodiment, six drug-sensitive paper strips 11 are stuck on the upper side surface of the bottom of the box body 10, and the volume of the box body is 30 mL.
1) 5 mL of morning sputum specimen of a patient is collected and digested with a mixed liquor of 2% of sodium hydroxide and 0.5% of NALC;
2) The morning sputum specimen is put into a centrifugal machine and centrifugally separated for 10 minutes at the rotational speed of 4,500 rpm, and the *mycobacterium tuberculosis* in the specimen is fully precipitated;
3) The supernatant fluid is removed to obtain enriched specimens, and then the specimen pretreatment is finished;
4) The enriched specimen is eluted with 10 mL of liquid medium to be prepared into a liquid culture specimen in the form of liquid bacterial suspension;
5) The liquid culture specimen is put into the incubator with temperature of 37° C for enriched culture, and the specific time of enriched culture is determined according to the amount of bacteria discharged of patients. If the detection result about the condition of a tuberculosis patient is ++++, the enriched culture need not be carried out;
6) The preparation of liquid drug-sensitivity test specimens for *mycobacterium tuberculosis:*
   (1) The temperature of the high-temperature zone of the heating thermostat unit is set to 90° C, and the temperature of the constant-temperature zone is set to 42° C;
   (2) The solid medium is put into the first hole for clamping a high-temperature cup 6 in the high-temperature zone of the heating thermostat unit to be heated and molten into liquid; the volume is 30 mL after the liquid culture specimen is mixed with the solid medium; the solid medium has the physical properties that the solid medium is molten into liquid from a solid state when the temperature rises to 90° C; the solid medium is solidified into solid from a liquid state after the temperature drops to 40° C;
   (3) The liquefied solid medium is put into the second hole for clamping a constant-temperature cup 8 in the constant-temperature balancing zone of the heating thermostat unit for cooling, and when the temperature of the liquefied solid medium drops to 42° C, the solid medium is still in a liquid state;
   (4) The liquid culture specimen after enriched culture is put into the second hole for clamping a constant-temperature cup 8 in the constant-temperature zone of the heating thermostat unit, so as to enable the temperature to rise to 42° C;
   (5) The liquefied solid medium with temperature of 42°C and the liquid culture specimen with temperature of 42° C are poured into a mixing cup to obtain liquid specimens for testing of drug sensitivity. To ensure the solid medium is in a liquid state during the mixing process, the mixing cup should be put into the third hole for clamping a mixing cup 3 in the constant-temperature zone of the heating thermostat unit for mixing.
7) The preparation of solid specimens for testing of drug sensitivity:
   (1) The box body 10 without the box cover 13 is put into the clamping groove of an inverted plate 2 in the constant-temperature zone of the heating thermostat unit, so as to enable the temperature to rise to 42° C;
   (2) The liquid specimens for testing of drug sensitivity are poured in the box body 10;
   (3) After the box cover 13 is closed, the culture box is taken out and placed at normal temperature, so that the liquid drug sensitivity test specimen IV is solidified and prepared into a solid drug sensitivity test specimen.
8) The solid drug sensitivity test specimen is put into an incubator with temperature of 37° C for culture, the color of the solid drug sensitivity test specimen is observed, and when its color is pink or light yellow, the next test step can be carried out;
9) After the box cover 13 is taken down, 1 mL of potassium tellurite as an indicator is dripped into the solid drug sensitivity test specimen, and then the box cover 13 is closed;
10) The solid drug sensitivity test specimen is put into the incubator with temperature of 37° C; the inhibition zone is observed, and the effect of drugs to bacteria is judged according to the size of the inhibition zone.

The liquid medium in the embodiment adopts 7H9.

The mixture ratio between the solid medium and liquid medium in the embodiment is to add a part of solid medium in every 100 mL of 7H9. Each part of the solid medium contains 75 mL of vitelline fluid, 4.6 g of soluble starch, 0.25 g of L-casein, 4 mL of phenol red, 1.25 g of agarose, 0.2 g of urea, and a proper amount of bacteriostat (malachite green).

Optionally, the indicator in the embodiment is sodium tellurite.

The agarose in the solid medium in the invention is used for solidifying the medium in drug sensitivity test. Agar can also be selected besides agarose.

The solid medium in the invention has the physical properties that the solid medium is solid at normal temperature; when the temperature rises below the melting temperature, the solid medium is still in the solid state, and after the temperature is higher than the melting temperature, the solid medium is molten into liquid; when the temperature drops above the solidification temperature, the solid medium is still in the liquid state, and after the temperature is lower than the solidification temperature, the solid medium is solidified into solid.

The selection criteria of melting temperature of the solid medium in the invention are as follows: 1. When the temperature of the solid medium rises to melting temperature, the nutrients are not destroyed; 2. When the solid drug sensitivity test specimen is put into the incubator with temperature of 37° C, it is still in the solid state; 3. The operation is convenient. Therefore, the melting temperature of the solid medium in the invention ranges from 50° C to 95° C.

The selection criteria of solidification temperature of the solid medium in the invention are as follows: 1. When the temperature of the solid medium drops below the solidification temperature, the *mycobacterium tuberculosis* is mixed with the solid medium, the temperature should be suitable for survival of *mycobacterium tuberculosis;* 2. The 37° C culture temperature of *mycobacterium tuberculosis* can be reached more quickly; 3. The operation is convenient. Therefore, the solidification temperature of the solid medium in the invention ranges from 25° C to 45° C. When the solidification temperature is too low and even lower than room temperature, especially when weather is hotter, the solid medium is required to be put into a refrigerating apparatus for solidification, thus the operation is inconvenient; meanwhile, when the temperature is far lower than the 37° C culture temperature of *mycobacterium tuberculosis,* the culture temperature of *mycobacterium tuberculosis* cannot be achieved as quickly as possible. If the solidification temperature is too high, it's not favorable for survival of *mycobacterium tuberculosis.*

The indicator in the invention can adopt a growth indicator or biochemical reaction indicator. The growth indicator should meet the requirements that the growth and reproduction of *mycobacterium tuberculosis* can be indicated, and the phenomenon of observable discoloration is formed; MTT, XTT, and Alamar-Blue are practicable. The biochemical reaction indicator should meet the requirements that the biochemical reaction indicator can take part in a biochemical reaction in specific biochemical reactions of *mycobacterium tuberculosis,* and the phenomenon of observable discoloration is formed; and urea, potassium tellurite, and sodium tellurite are practicable.

The indicator in the embodiment above adopts urea and potassium tellurite, and besides, one of urea, MTT, XTT, and Alamar-Blue can be selected as the indicator. The test process only comprises 8 steps, wherein the first seven are the same, and the last one is that the solid drug sensitivity test specimen is put into the incubator with temperature of 37° C for culture, the inhibition zone is observed, and the effect of drugs to bacteria is further judged according to the size of the inhibition zone.

## Claims

1. A method for testing drug sensitivity of *mycobacterium tuberculosis,* the method comprising the following steps:
a) pre-treating target specimens to obtain enriched specimens;
b) adding a liquid medium to the enriched specimens to obtain liquid culture specimens, and performing enriched culture for 0-7 days;
c) heating and melting a solid medium to obtain a liquefied solid medium, and reducing the temperature of the liquefied solid medium to the corresponding culture temperature of the liquid culture specimens or room temperature, the solid medium having a melting temperature of 50-95°C and a solidification temperature of 25-45°C, the solid medium comprising vitelline, soluble starch, L-casein, phenol red, agar or agarose, bacteriostat, and urea;
d) mixing the liquid culture specimens with the liquefied solid medium to obtain liquid specimens for testing of drug sensitivity;
e) solidifying the liquid specimens for testing of drug sensitivity to yield solid specimens for testing of drug sensitivity with antibiotic discs;
f) cultivating the solid specimens for testing of drug sensitivity until the solid specimens turn pink or light yellow; and
g) dripping an indicator to the solid specimens when the solid specimens turn pink or light yellow in step f), observing a drug inhibition zone through a visible colour change of the indicator and determining the drug sensitivity of *mycobacterium tuberculosis.*

2. The method of claim 1, **characterized in that** the indicator is a growth indicator or biochemical reaction indicator of *mycobacterium tuberculosis.*

3. The method of claim 2, **characterized in that** the indicator is a tellurite, a mixture of a tellurite and urea, urea, 3-(4,5-dimethylthiazol-2-yl)-2,5-diphenyltetrazolium bromide, 2,3-bis-(2-methoxy-4-nitro-5-sulfophenyl)-2H-tetrazolium-5-carboxanilide, or Alamar-Blue.

4. The method of claim 3, **characterized in that** in step g), as the indicator the tellurite is dripped into the solid specimens.

5. The method of claim 1, wherein in step g) the indicator is a tellurite; and the tellurite is dripped into the solid specimens for testing of drug sensitivity.

6. The method of claim 5, **characterized in that** the tellurite is sodium tellurite or potassium tellurite.

## Patentansprüche

1. Verfahren zum Prüfen derArzneimittelempfindlichkeit von *Mycobacterium tuberculosis,* wobei das Verfahren die folgenden Schritte umfasst:
a) Vorbehandeln von Zielproben, um angereicherte Proben zu gewinnen;
b) Zugeben eines flüssigen Mediums zu den angereicherten Proben, um flüssige Kulturproben zu gewinnen, und Durchführen angereicherter Kultur für 0 bis 7 Tage;
c) Erhitzen und Schmelzen eines festen Mediums, um ein verflüssigtes festes Medium zu gewinnen, und Senken der Temperatur des verflüssigten festen Mediums auf die entsprechende Kulturtemperatur der flüssigen Kulturproben oder auf Raumtemperatur, wobei das feste Medium eine Schmelztemperatur von 50 bis 95 °C und eine Erstarrungstemperatur von 25 bis 45 °C aufweist und das feste Medium Vitellin, lösliche Stärke, L-Casein, Phenolrot, Agar oder Agarose, Bakteriostat und Harnstoff umfasst;
d) Mischen der flüssigen Kulturproben mit dem verflüssigten festen Medium, um flüssige Proben zum Prüfen der Arzneimittelempfindlichkeit zu gewinnen;
e) Verfestigen der flüssigen Proben zum Prüfen der Arzneimittelempfindlichkeit, um feste Proben zum Prüfen der Arzneimittelempfindlichkeit mit antibiotischen Scheiben zu gewinnen;
f) Kultivieren der festen Proben zum Prüfen der Arzneimittelempfindlichkeit, bis die festen Proben rosafarben oder hellgelb werden; und
g) Tropfen eines Indikators zu den festen Proben, wenn die festen Proben in Schritt f) rosafarben oder hellgelb werden, Beobachten einer Arzneimittelhemmzone durch eine sichtbare Farbänderung des Indikators und Bestimmen der Arzneimittelempfindlichkeit von *Mycobacterium tuberculosis.*

2. Verfahren nach Anspruch 1, **dadurch gekennzeichnet, dass** der Indikator ein Wachstumsindikator oder ein biochemischer Reaktionsindikator von *Mycobacterium tuberculosis* ist.

3. Verfahren nach Anspruch 2, **dadurch gekennzeichnet, dass** der Indikator ein Tellurit, ein Gemisch aus einem Tellurit und Harnstoff, Harnstoff, 3-(4,5-Dimethylthiazol-2-yl)-2,5-diphenyltetrazoliumbromid, 2,3-Bis-(2-methoxy-4-nitro-5-sulfophenyl)-2H-tetrazolium-5-carboxanili d oder Alamarblau ist.

4. Verfahren nach Anspruch 3, **dadurch gekennzeichnet, dass** in Schritt g) das Tellurit als der Indikator in die festen Proben getropft wird.

5. Verfahren nach Anspruch 1, wobei der Indikator in Schritt g) ein Tellurit ist und das Tellurit in die festen Proben zum Prüfen der Arzneimittelempfindlichkeit getropft wird.

6. Verfahren nach Anspruch 5, **dadurch gekennzeichnet, dass** das Tellurit Natriumtellurit oder Kaliumtellurit ist.

## Revendications

1. Méthode pour tester la sensibilité d'un médicament pour *mycobacterium tuberculosis,* la méthode comprenant les étapes suivantes consistant à :
a) prétraiter des échantillons cibles pour obtenir des échantillons enrichis ;
b) ajouter un milieu liquide aux échantillons enrichis pour obtenir des échantillons de culture liquides, et réaliser une culture enrichie pendant 0 à 7 jours ;
c) chauffer et faire fondre un milieu solide pour obtenir un milieu solide liquéfié, et réduire la température du milieu solide liquéfié à la température de culture correspondante des échantillons de culture liquides ou à la température ambiante, le milieu solide ayant une température de fusion de 50 à 95 °C et une température de solidification de 25 à 45 °C, le milieu solide comprenant de la vitelline, de l'amidon soluble, de la L-caséine, du rouge de phénol, de la gélose ou de l'agarose, un bactériostatique et de l'urée ;
d) mélanger les échantillons de culture liquides avec le milieu solide liquéfié pour obtenir des échantillons liquides pour tester la sensibilité d'un médicament ;
e) solidifier les échantillons liquides pour tester la sensibilité d'un médicament pour donner des échantillons solides afin de tester la sensibilité d'un médicament avec des disques antibiotiques ;
f) cultiver les échantillons solides pour tester la sensibilité d'un médicament jusqu'à ce que les échantillons solides deviennent rose ou jaune clair ; et
g) faire tomber goutte à goutte un indicateur sur les échantillons solides lorsque les échantillons solides deviennent rose ou jaune clair dans l'étape f), observer une zone d'inhibition de médicament par un changement de couleur visible de l'indicateur et déterminer la sensibilité d'un médicament pour *mycobacterium tuberculosis.*

2. Méthode selon la revendication 1, **caractérisée en ce que** l'indicateur est un indicateur de croissance ou un indicateur de réaction biochimique de *mycobacterium tuberculosis.*

3. Méthode selon la revendication 2, **caractérisée en ce que** l'indicateur est une tellurite, un mélange d'une tellurite et d'une urée, une urée, le bromure de 3-(4,5-diméthylthiazol-2-yl)-2,5-diphényltétrazolium, le 2,3-bis-(2-méthoxy-4-nitro-5-sulfophényl)-2H-tétrazolium-5-carboxanilid e, ou le bleu Alamar.

4. Méthode selon la revendication 3, **caractérisée en ce que** dans l'étape g), en tant qu'indicateur, de la tellurite est déposée goutte à goutte dans les échantillons solides.

5. Méthode selon la revendication 1, dans laquelle dans l'étape g), l'indicateur est une tellurite ; et la tellurite est déposée goutte à goutte dans les échantillons solides pour tester la sensibilité d'un médicament.

6. Méthode selon la revendication 5, **caractérisée en ce que** la tellurite est la tellurite de sodium ou la tellurite de potassium.
